# EUROPEAN PATENT APPLICATION

(11) **EP 4 767 980 A1**
(43) Date of publication of application: **01.07.2026**
(21) Application number: 24867597.7
(22) Date of filing: 23.09.2024
(51) Int. Cl.: A61B 34/20, A61B 17/3203, A61B 8/08

(54) **ULTRASONIC IMAGE-BASED APPARATUS FOR MONITORING POSITIONAL DEVIATION OF SURGICAL INSTRUMENT**

(30) Priority: 22.09.2023 CN 202311226358
(71) Applicant: Healinno (Beijing) Medical Technology Co., Ltd., Beijing 100176 (CN)
(72) Inventor: CHEN, Wenbo, Beijing 100176 (CN); SHI, Ce, Beijing 100176 (CN); MA, Tao, Beijing 100176 (CN); ZHAO, Jing, Beijing 100176 (CN)
(74) Representative: Metida
(86) International application number: PCT/CN2024/120346
(87) International publication number: WO 2025/061192

(57) **Abstract**

An ultrasonic image-based apparatus for monitoring positional deviation of a surgical instrument, comprising a surgical instrument (112), an ultrasonic subsystem comprising an ultrasonic probe (122), and a control subsystem comprising a processor. The control subsystem is separately connected to the surgical instrument (112) and the ultrasonic subsystem, and the ultrasonic subsystem continuously acquires ultrasonic images of a target tissue site (200) and transmits the acquired series of ultrasonic image frames to the processor of the control subsystem. The surgical instrument (112) moves in the target tissue site (200) according to a surgical planning path set by the processor. The tail end of the surgical instrument (112) is provided with an arc-shaped top end portion (22). The processor comprises an image processing program. The image processing program performs calculation on the basis of the series of ultrasonic image frames, so as to determine a deviation between the position of a tracking sub-region in a current image frame and that of the tracking sub-region in a previous image frame, the tracking sub-region being a region comprising the arc-shaped top end portion (22) in the ultrasonic image. The apparatus can implement accurate, real-time, and effective monitoring of the displacement of the surgical instrument (112) on the basis of the ultrasonic image, ensuring surgical safety.

## Description

### Technical Field

This application relates to the field of medical instruments, in particular to an ultrasonic image-based apparatus for monitoring positional deviation of a surgical instrument, and further, particularly relates to displacement monitoring for a waterjet during a robotic-assisted waterjet minimally invasive surgery procedure.

### Background Art

At present, in the field of medical instruments, technologies for automated surgical schemes such as surgical planning and navigation are developing rapidly. Generally, after a surgical planning scheme is formulated, a surgical instrument moves under the drive of a surgical robot according to the planning scheme, thereby completing the surgical scheme along a planned path. In such surgery, displacement monitoring for the surgical instrument is particularly important. Once a surgical instrument executing the surgery undergoes an unnecessary positional deviation, it means that the surgical instrument cannot complete the surgery along the planned path or even if the surgery is completed, the actual result will deviate significantly from a preset plan. A minor error, even a slight positional deviation, can lead to a significant reduction in surgical precision.

Taking a waterjet minimally invasive surgery system based on surgical planning as an example, the waterjet minimally invasive surgery system includes a waterjet subsystem, an ultrasonic device, and a corresponding control system. During procedures such as waterjet surgery on prostate tissue areas, under the guidance of a rectal ultrasonic probe, a mechanical arm is used to insert a waterjet into a target tissue site. After the waterjet is in place, a host computer software system plans a scanning path and a water jet intensity of the waterjet, and the mechanical arm guides the waterjet to perform a surgical operation using planned parameters. Throughout the entire process from planning to the end of the surgery, the positions of the waterjet, the ultrasonic probe, and a patient must remain unchanged. Otherwise, surgical planning parameters will be invalid and accidents may occur during the operation.

However, such positional deviation is inevitable in the surgical procedure. For example, unnecessary movement of a patient or an operator or other surrounding environments may cause displacement of the surgical instrument, which makes it difficult to continue the planning scheme. Therefore, in order to ensure the accuracy and safety of surgery, the displacement monitoring for the surgical instruments is important and a key factor determining the quality of surgery.

In the prior art, a common displacement monitoring approach is to calibrate and record spatial positions of a waterjet and an ultrasonic probe by using optical and/or magnetic navigation. Such methods require additional sensors or markers as well as pre-calibration processes to monitor movement of the spatial positions of the waterjet and the ultrasonic probe, which leads to high costs and other problems such as disinfection process for additional markers, resulting in numerous inconveniences.

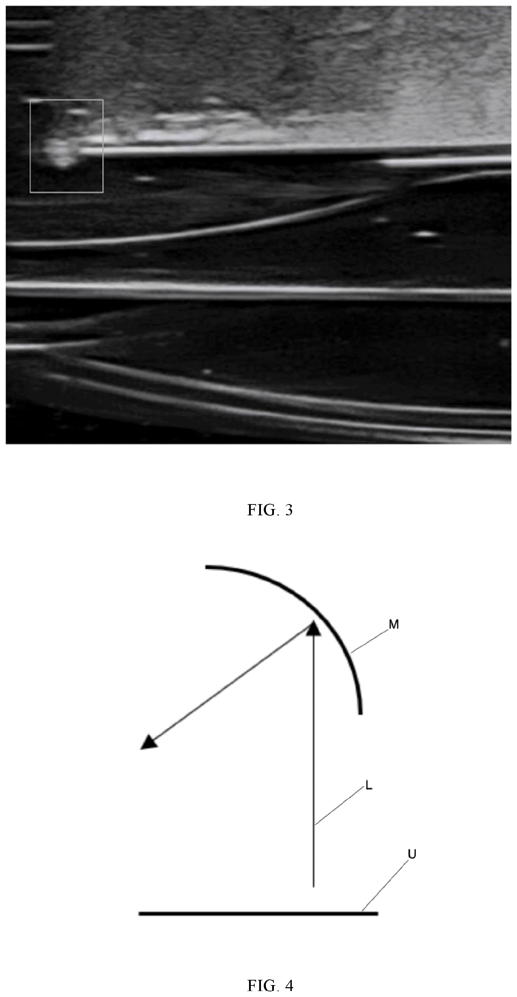

Some researchers have also proposed a approach of using images to monitor displacement. However, the solutions based on this approach formidable challenges. On one hand, imaging means such as X-ray, CT and MRI, which can obtain high-definition images, are difficult to be directly applied during surgery for acquiring real-time images due to factors like radiation from the equipment, bulky device size, and slow imaging processes, let alone monitoring real-time displacement. On the other hand, an ultrasonic image is a commonly used imaging means in surgery. However, it is very difficult to perform real-time recognition of surgical instruments in an ultrasonic image due to the inherent imaging quality of the ultrasonic image. Taking a waterjet minimally invasive surgery system as an example, a waterjet serving as a surgical instrument appears as a long straight line in an ultrasonic image, and a sheath usually serving as an insertion auxiliary instrument or an endoscope for auxiliary diagnosis also appears as a long straight line close to the waterjet (as shown in Fig. 3), which makes it easy to form a large number of artifacts during ultrasound imaging, making displacement monitoring nearly impossible.

### Summary of the Invention

This application provides an ultrasonic image-based apparatus for monitoring positional deviation of a surgical instrument, the apparatus including: a surgical instrument; an ultrasonic subsystem including an ultrasonic probe; and a control subsystem including a processor, the control subsystem being separately connected to the surgical instrument and the ultrasonic subsystem, the surgical instrument moving in a target tissue site according to a surgical planning path set by the processor, in which a tail end of the surgical instrument is provided with an arc-shaped top end portion including a curved outer surface and a curved inner surface, the inner surface is an arc-shaped surface relatively closer to a jetting region of the surgical instrument, and the outer surface is an arc-shaped surface relatively farther from the jetting region of the surgical instrument; after the surgical instrument is in place, the ultrasonic probe starts to continuously acquire ultrasonic images of the target tissue site and transmit acquired series of ultrasonic image frames to the processor of the control subsystem, the processor includes an image processing program configured to perform calculation based on the series of ultrasonic image frames to determine a positional deviation between a tracking sub-region in a current frame image and a tracking sub-region in a previous frame image, the tracking sub-region is a local region including the arc-shaped top end portion in the ultrasonic images, and the image processing program extracts image features of the tracking sub-region by a feature extraction method to obtain image features of the arc-shaped top end portion for subsequent displacement tracking; and the surgical instrument includes a waterjet and a sheath including an elongated support body, and the arc-shaped top end portion extends from a distal end of the elongated support body, or the surgical instrument includes a waterjet including an elongated cutter tube and a cutter head located at a distal end of the cutter tube, and the cutter head has an arc shape and forms the arc-shaped top end portion.

Preferably, the apparatus further includes a fixed base connected to the surgical instrument through a first mechanical arm and a first adapter and connected to the ultrasonic probe through a second mechanical arm and a second adapter, the ultrasonic probe being a transrectal ultrasonic probe.

Preferably, the inner surface and the outer surface together form an arc-shaped top end portion with a bending angle of 30° to 75°.

Preferably, the arc-shaped top end portion includes tungsten, and/or the arc-shaped top end portion comprises a smooth outer surface and a rough inner surface, and the rough inner surface is formed by embedding irregular concave-convex pits, grooves, or steps, or by frosting.

Preferably, a distance between the ultrasonic probe and the sheath is R, and a curvature of the arc-shaped top end portion is K=1/R.

Preferably, the tracking sub-region is a smallest image region including all arc-shaped top end portions.

Preferably, the apparatus alarms or pauses a surgery when the monitored positional deviation exceeds a set safety displacement distance.

Preferably, the image processing program further performs steps of: identifying and marking a tracking sub-region on an initial frame image; updating the tracking sub-region in each subsequent frame image; and calculating a positional deviation between the tracking sub-region in the current frame image and an estimated tracking sub-region, where a position of the estimated tracking sub-region is a position of the tracking sub-region in the previous frame image or a position obtained by superimposing a planned movement path between two frames onto the position of the tracking sub-region in the previous frame image.

Preferably, the image processing program further performs steps of: setting center position coordinates of the tracking sub-region on the initial frame image as an original coordinate origin for tracking; subsequently updating the tracking sub-region in each subsequent frame image and calculating center position coordinates of the updated tracking sub-region; and calculating a deviation between center position coordinates of the tracking sub-region in the current frame image and center position coordinates of the estimated tracking sub-region.

Preferably, the image processing program further displays boundary points or center position points of the tracking sub-region in the current frame image in different colors and/or prompts textual information thereof based on a magnitude of the positional deviation.

Preferably, the image processing program performs identification and tracking based on a small sample of the tracking sub-region.

Preferably, the image processing program further performs steps of: constructing an offline feature template library based on the tracking sub-region; determining an offline feature template f1(x) that best matches the initial frame image and the tracking sub-region in the initial frame image; determining a best feature template F(x) for a next frame image according to each current frame image; and determining a tracking sub-region in the next frame image based on the determined best feature template F(x).

Preferably, the step of constructing an offline feature template library based on the tracking sub-region includes a step of offline acquiring an original feature template of the tracking sub-region, the original feature template being a combination of ultrasonic images and feature information of the tracking sub-region.

Preferably, the step of constructing an offline feature template library based on the tracking sub-region further includes a step of constructing an offline feature template library based on the original feature template, the offline feature template library including a plurality of feature templates generated based on the original feature template.

Preferably, the offline feature template library includes offline feature templates with different depth parameters and offline feature templates of different scales formed by determining scale factors based on the different depth parameters.

Preferably, the step of determining an offline feature template f1(x) that best matches the initial frame image and the tracking sub-region in the initial frame image includes: after both the ultrasonic probe and the surgical instrument are in place, starting the ultrasonic probe to acquire the initial frame image; selecting, based on a depth parameter of the ultrasonic probe, a plurality of candidate feature templates corresponding to the depth parameter from the offline feature template library; and calculating a matching degree between the initial frame image and each of the plurality of candidate feature templates one by one, taking a feature template having a highest matching degree as the best matching offline feature template f1(x), and taking a region having a highest matching degree as the tracking sub-region in the initial frame image.

Preferably, in the step of determining a best feature template F(x) for a next frame image according to each current frame image, the best feature template F(x) for the next frame image is determined at least based on an image feature of the tracking sub-region in the feature template f1(x) and an image feature of the tracking sub-region in the current frame image.

Preferably, in the step of determining a best feature template F(x) for each next frame image based on each current frame image, based on a feature of the tracking sub-region in the feature template f1(x) selected from the offline feature template library, a feature of the tracking sub-region in the current frame image, and a deep feature of the tracking sub-region in the current frame image extracted by a pre-trained deep learning neural network, the best feature template F(x) for the next frame image is derived by weighted fusion.

Preferably, a window size of the tracking sub-region is adaptively adjustable.

Preferably, a current ultrasonic depth parameter is acquired in real time and determined, and when the depth parameter changes between two adjacent frames of ultrasonic images, scale factors in a parameter set are adaptively updated according to a scale factor set corresponding to a set depth.

The technical scheme provided by this application can solve the above-mentioned technical problem and has at least one of the following technical effects. The technical scheme can overcome a technical difficulty in monitoring a surgical instrument based on an ultrasonic image, so that accurate, real-time and effective monitoring of displacement of the surgical instrument based on the ultrasonic image becomes possible. Compared with conventional displacement monitoring methods, the technical scheme significantly reduces costs and eliminates cumbersome operation steps such as disinfecting additional markers. The displacement of the surgical instrument can be monitored using an intraoperative ultrasonic imaging subsystem built-into a surgical system, and monitoring results can be fed back and prompted in real time, facilitating the operation for an operator or a doctor. Meanwhile, accurate and real-time displacement monitoring facilitates the safe implementation of a surgical scheme in strict accordance with the surgical planning, improves the quality of surgery, and reduces medical accidents. In addition, the displacement monitoring information may also be directly used to adjust a surgical planning scheme, which is beneficial for precise control of an automated surgical procedure.

### Descriptions of the Drawings

The drawings described herein are used to provide further understanding of this application, and constitute a part of this application. The schematic embodiments of this application and descriptions thereof are intended to explain this application, but do not constitute an improper limitation on this application. In the drawings:
FIG. 1 is a schematic structural diagram of a waterjet minimally invasive surgery system according to an embodiment of this application;
FIG. 2 is a schematic diagram of spatial distribution of an ultrasonic probe and a surgical instrument according to the embodiment of this application;
FIG. 3 is an ultrasonic image of a conventional waterjet and a target tissue site according to the background art;
FIG. 4 is a schematic diagram of ultrasonic reflection by an arc-shaped top end portion of the surgical instrument according to the embodiment of this application;
FIG. 5 is a schematic diagram of a main structure of a waterjet subsystem according to the embodiment of this application;
FIG. 6 is a schematic diagram of ultrasonic reflection by a rough inner surface of the arc-shaped top end portion of the surgical instrument according to the embodiment of this application;
FIG. 7 is an ultrasonic image including a tracking sub-region according to the embodiment of this application;
FIG. 7a is a local ultrasonic image including the tracking sub-region according to the embodiment of this application;
FIG. 7b is a schematic diagram of an image feature of the tracking sub-region after feature extraction according to the embodiment of this application;
FIG. 8 is a schematic diagram of tracking displacement of the surgical instrument based on the tracking sub-region according to the embodiment of this application;
FIG. 9 is a schematic flowchart of template determination and update for the tracking sub-region according to the embodiment of this application; and
FIG. 10 is a schematic diagram of a system workflow when an apparatus for monitoring displacement of the surgical instrument according to the embodiment of this application is used in a surgery system.

### Detailed Description

In order to make the object, technical solutions and advantages of this application clearer, the technical solutions of this application will be described clearly and completely with reference to specific embodiments of this application and corresponding drawings. It is apparent that the described embodiments are merely some, but not all, of the embodiments of this application. Based on the embodiments in this application, all other embodiments obtained by those skilled in the art without creative work fall within the protection scope of this application.

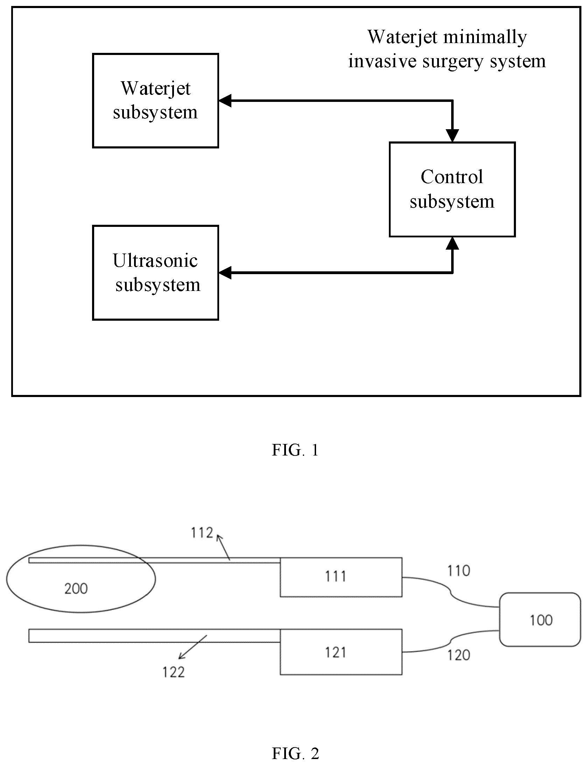

An ultrasonic image-based apparatus for monitoring displacement of a surgical instrument according to an embodiment of this application includes: a surgical instrument, an ultrasonic subsystem, and a control subsystem. The ultrasonic subsystem includes an ultrasonic probe, the control subsystem includes a processor, the control subsystem is separately connected to the surgical instrument and the ultrasonic subsystem, the ultrasonic subsystem continuously acquires ultrasonic images of a target tissue site and transmits acquired series of ultrasonic image frames to the processor of the control subsystem, and the surgical instrument moves in the target tissue site according to a surgical planning path set by the processor. Taking a waterjet minimally invasive surgery system as an example, referring to FIG. 1, the waterjet minimally invasive surgery system includes: a waterjet subsystem, an ultrasonic subsystem, and a control subsystem. The control subsystem is separately connected to the ultrasonic subsystem and the waterjet subsystem, and the control subsystem can obtain image data from the ultrasonic subsystem and control parameter data from the waterjet subsystem, and also issue adjustment instructions to adjust imaging parameters of the ultrasonic subsystem and control parameters of the waterjet subsystem. The ultrasonic subsystem includes an ultrasonic probe. When used for surgeries related to prostate tissue, transrectal ultrasound (TRUS) is preferred. The ultrasonic subsystem continuously acquires ultrasonic images of a target tissue site and transmits the acquired ultrasonic images to a processor in the control subsystem. The control subsystem includes a processor configured to receive an image from the ultrasonic subsystem and analyze the image.

FIG. 2 is a schematic diagram of spatial arrangement of an ultrasonic probe and a surgical instrument according to the embodiment of this application. A fixed base 100 serving as a fixed reference component is connected to a surgical instrument 112 through a first mechanical arm 110 and a first adapter 111, and is connected to an ultrasonic probe 122 through a second mechanical arm 120 and a second adapter 121.

When used for surgeries related to prostate tissue, the surgical instrument 112 moves to a target tissue site 200 through the urethra or perineum. The transrectally inserted ultrasonic probe 122 is oriented toward the urethra or perineum where the surgical instrument 112 is located to acquire an ultrasonic image of the target tissue site 200.

As mentioned in the background art, although images of the surgical instrument and target tissue site can be acquired in real time using the ultrasonic subsystem, the image clarity is limited. Therefore, conventional surgical planning relies on preoperative MR or CT images. Intraoperative ultrasonic images are usually used only for understanding a general situation during surgery and are only available to doctors who have knowledge and experience about ultrasonic images of target tissues. In particular, in a case where the surgical instrument is a waterjet, since the waterjet and a sheath assisting the waterjet entry into the target tissue site are both linear structures, there are many artifacts in an ultrasonic image near the waterjet. As shown in FIG. 3, which is an ultrasonic image of a conventional waterjet and a target tissue site according to the background art, the location of the waterjet cannot be clearly determined from the ultrasonic image alone, let alone displacement monitoring of the waterjet, especially displacement monitoring of an energy jetting port at a tail end of the waterjet.

To solve the problem, this application first focuses on improving the design of a shape of a tail end of the surgical instrument, configuring the tail end of the surgical instrument to include an arc-shaped top end portion. As shown in FIG. 4, ultrasonic waves emitted by an ultrasonic probe U are reflected back to the ultrasonic probe U by the arc-shaped top end portion M. More ultrasonic reflection beams L are reflected back to the ultrasonic probe U than that of a linear structure, thereby enhancing the identifiability of this part in the ultrasonic image to some extent.

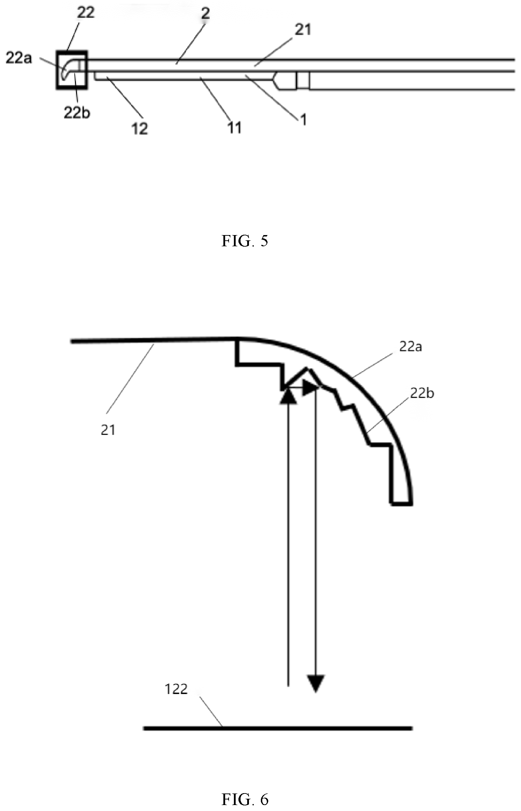

Referring to FIG. 5, which is a schematic diagram showing a main structure of the waterjet subsystem in a waterjet displacement monitoring apparatus according to an embodiment of this application, in this embodiment, the surgical instrument includes a waterjet 1 and a sheath 2, the sheath 2 is inserted into a target tissue site and includes an elongated support body 21, and an arc-shaped top end portion 22 extends from a distal end of the elongated support body 21. The waterjet 1 moves along a channel defined by the sheath 2 according to the surgical planning path set by the processor. The sheath 2 is inserted into a target tissue site and can provide the function of fixing and/or supporting surrounding tissues. The waterjet 1 includes a cutter tube 11 and a cutter head 12, the cutter tube 11 and the cutter head 12 move along the channel defined by the sheath 2 according to the surgical planning path set by the processor. Preferably, the arc-shaped top end portion 22 is further provided with a suction through hole communicating with a suction channel inside the support body 21.

In an alternative embodiment, the waterjet 1 is provided with an arc-shaped top end portion. That is, the surgical instrument includes a waterjet including the elongated cutter tube 11 and the cutter head 12 located at a distal end of the cutter tube 11, and the cutter head 12 has an arc shape and forms the arc-shaped top end portion.

As shown in FIG. 5, the arc-shaped top end portion 22 includes a curved outer surface 22a and a curved inner surface 22b. In an alternative embodiment, when the cutter head of the waterjet is configured to have an arc shape, the cutter head 12 includes a curved outer surface 12a and a curved inner surface 12b (not shown). The inner surface is an arc-shaped surface relatively closer to a jetting region of the waterjet, and the outer surface is an arc-shaped surface relatively farther from the jetting region of the waterjet. The outer surface is curved and smooth, which can further facilitate the reduction of trauma to surrounding tissues during insertion of the surgical instrument into the target tissue site, thereby providing better protection for the surrounding tissues. The inner surface has a curved arc shape matching the outer surface, and together with the outer surface, forms an arc-shaped top end portion. In order to achieve better protective effect during insertion into urethra, a bending angle is preferably 30° to 75°. Compared with the conventional linear surgical instrument (waterjet or sheath), the arc-shaped tail end of the surgical instrument is beneficial to provide better identifiability in the ultrasonic image.

The arc-shaped top end portion may include tungsten, and it is advantageous to make the arc-shaped top end portion from a tungsten material or to add a tungsten-containing coating on the outer surface and/or inner surface of the arc-shaped top end portion. Furthermore, not limited to the arc-shaped top end portion, the entire surgical instrument may also be made of a tungsten-containing material. Since the acoustic impedance of tungsten is about 70 times that of human soft tissue, the identifiability of the arc-shaped top end portion 22 containing tungsten in an ultrasonic image can also be improved.

As shown in FIG. 6, when the inner surface is a rough surface, the rough surface may be formed by embedding a large number of irregular concave-convex pits, irregular grooves, or steps, or by frosting. The inner surface is set to be rough, especially the rough surface includes an irregular structure which includes a random combination of acute angle surfaces and obtuse angle surfaces. The irregular structure is beneficial to improving identifiability of the arc-shaped top end portion in ultrasonic images because the rough arc surface can enhance ultrasonic waves reflected into a sensor of the ultrasonic probe while suppressing or reducing ultrasonic waves reflected to other directions.

In a preferred embodiment, the ultrasonic probe in the ultrasonic subsystem is a convex array probe. A curvature K of the arc-shaped top end portion is related to a distance R between the ultrasonic probe and the sheath, and the calculation formula for the curvature K is: K = 1/R.

In the scenario of waterjet surgery on prostate tissue, if a distance from the urethra or perineum (position of the sheath) to the rectum (position of the ultrasonic probe) after insertion of the waterjet is R, the curvature K of the arc-shaped top end portion is preferably set as 1/R. The arc-shaped top end portion with such a range of curvature can provide better reflection and development effects in the ultrasonic image and thus provide better identifiability.

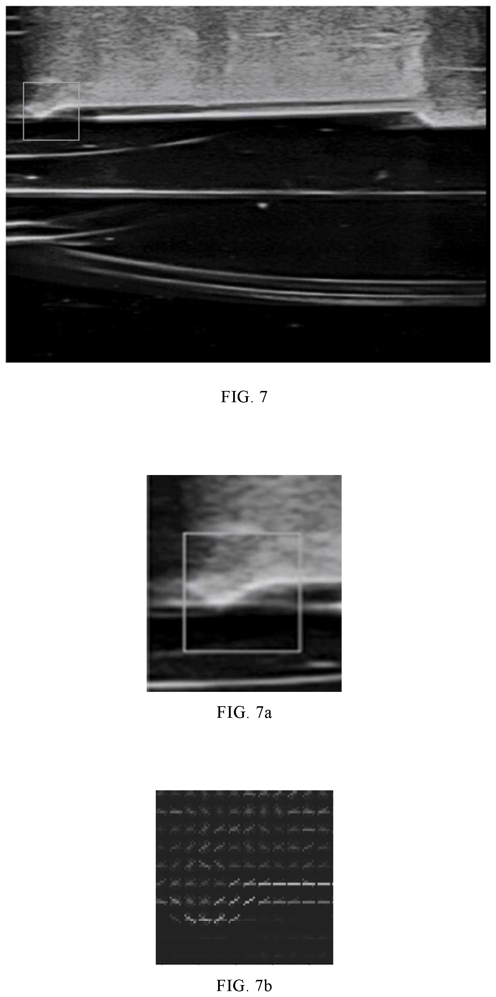

With the above one or more of the arrangements, the identifiability of the surgical instrument in the ultrasonic image can be greatly improved. The ultrasonic image obtained in a certain embodiment is shown in FIG. 7. In FIG. 7, a contour of the arc-shaped top end portion 22 can be clearly seen within a rectangular box, thereby making it possible to extract an image feature based on the contour within the rectangular box region and perform tracking based on the extracted feature.

In the waterjet subsystem shown in FIG. 5, the waterjet moves along a planned path, and a movement mode thereof typically includes two types.

Movement Mode 1: The sheath and the waterjet are driven by the first mechanical arm 110 or a similar component to move as a whole to a first target position, or return from the first target position to an initial position. In this case, the relative position between the sheath and the waterjet is fixed. The relative position between the waterjet and the arc-shaped top end portion of the sheath is also fixed. The waterjet has a fixed position with respect to the arc-shaped top end portion, but the waterjet may be displaced due to the movement of the whole sheath, for example. Therefore, the displacement of the arc-shaped top end portion is equal to the displacement of the waterjet.

Movement Mode 2: The sheath remains fixed at the first target position, and the waterjet moves to a second target position along a path defined by the sheath or returns from the second target position to the first target position. In this case, the movement of the waterjet relative to the sheath is the movement when the control subsystem drives the waterjet to execute an ablation/cutting operation under the guidance of the surgical planning scheme. A movement distance thereof may be calculated according to the surgical planning scheme and/or waterjet control parameters. At this time, the displacement of the waterjet includes both displacement caused by unnecessary movement of the sheath and displacement caused by a deviation in the waterjet control parameters. Since the deviation in waterjet control parameters can be timely detected and corrected by using PID feedback control, etc., it may be ignored here. Therefore, the displacement of the arc-shaped top end portion can still represent the displacement of the waterjet.

Based on the above considerations, this application proposes an approach of tracking the displacement of the waterjet by tracking the displacement of the arc-shaped top end portion, and further improves a tracking effect by specifically optimizing a tracking scheme.

In this application, an image region within a rectangular box shown in FIG. 7 is defined as a tracking sub-region. In this application, the tracking sub-region refers to a region including the arc-shaped top end portion in the ultrasonic image. The tracking sub-region is preferably a partial region of the entire ultrasonic image. Preferably, a minimum image region including all the arc-shaped top end portions may be selected. By selecting a smaller region as the tracking sub-region, a computational load for tracking can be reduced greatly and a processing speed is improved, so that real-time tracking can be achieved.

By improving the structure of the surgical instrument, the tracking sub-region where the arc-shaped top end portion is located is a region with the best identifiability in the ultrasonic image of the target tissue site, which provides a possibility for image recognition and image tracking. The sub-region includes features of the arc-shaped top end portion, and the displacement of the surgical instrument can be tracked by tracking the displacement of the arc-shaped top end portion.

FIG. 7a is a local original ultrasonic image including the tracking sub-region, and a region within a rectangular box in the figure is the tracking sub-region. Image features of the tracking sub-region are extracted using a feature extraction method, such as the histogram of oriented gradients, to obtain stable and clear image features of the arc-shaped top end portion, which are used for subsequent displacement tracking, as shown in FIG. 7b.

After the surgical instrument is in place (for example, after the sheath 2 and the waterjet 1 are moved as a whole to the first target position), the ultrasonic probe starts to acquire a series of ultrasonic image frames. The acquired first ultrasonic image is called an initial frame image. An image that undergoes specific image analysis and processing for each subsequent frame is called a current frame image. An image immediately before the current frame image is called a previous frame image, and an image immediately after the current frame is called a next frame image.

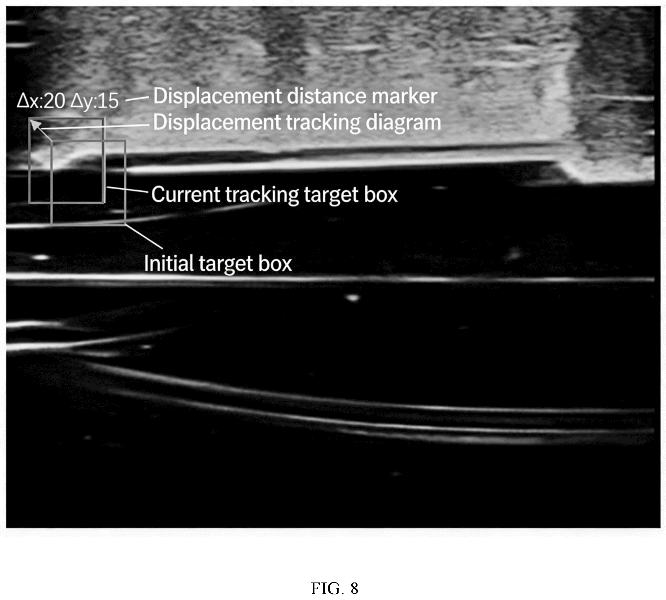

FIG. 8 is a schematic diagram of tracking displacement of the region based on the tracking sub-region. The ultrasonic probe continuously acquires a series of ultrasonic image frames. A right rectangular box represents a position of the tracking sub-region determined according to the initial frame image, a left rectangular box represents a position of the tracking sub-region on the current frame image, an arrow represents a direction of positional deviation of the tracking sub-region, and specific coordinate values of the positional deviation are annotated above the left rectangular box.

According to a preferred embodiment, a boundary or a center position of the tracking sub-region in the previous frame image may be marked with a preset original color value in the current frame image.

Boundary points or center position points of the tracking sub-region in the current frame image may also be displayed in different colors according to a magnitude of the positional deviation.

Considering the resolution of ultrasonic image and the influence of displacement of surgical instrument on human tissue, a displacement safety distance may be set. For example, in the scenario of waterjet surgery on prostate, the displacement safety distance is set to 5 mm. When the monitored positional deviation is within 0 mm to 5, the boundary points or center position points of the tracking sub-region in the current frame image are displayed in orange. When the monitored positional deviation is more than 5 mm, the boundary points or center position points of the tracking sub-region in the current frame image are displayed in red.

Preferably, the numerical value of the positional deviation may also be displayed simultaneously with the current frame image.

Preferably, a predetermined safety strategy may be executed according to the positional deviation. For example, when it is determined that the positional deviation exceeds a preset safety displacement distance threshold, an alarm and/or a surgical pause are performed.

A displacement monitoring method according to the embodiment of this application includes first identifying and marking a tracking sub-region on an initial frame image, updating the tracking sub-region in each subsequent frame image, determining a tracking sub-region of a current frame, calculating a positional deviation between the tracking sub-region in the current frame image and an estimated tracking sub-region, and using the positional deviation as a displacement value of the waterjet.

Preferably, the method further includes setting center position coordinates of the tracking sub-region on the initial frame image as an original coordinate origin for tracking, subsequently updating the tracking sub-region in each subsequent frame image and calculating center position coordinates of the updated tracking sub-region, calculating a positional deviation between center position coordinates of the tracking sub-region in the current frame image and center position coordinates of the estimated tracking sub-region, and using the positional deviation as a displacement value of the waterjet.

When the arc-shaped top end portion is a position-fixing component (such as the sheath) during the surgical procedure, a position of the estimated tracking sub-region is a position of the tracking sub-region in the previous frame image.

When the arc-shaped top end portion is a moving component (such as the waterjet cutter head) during the surgical procedure, the position of the estimated tracking sub-region is a position obtained by superimposing a planned movement path between two frames onto the position of the tracking sub-region in the previous frame image.

As described above, it is difficult to perform real-time identification on the surgical instrument in the ultrasonic image due to excessive artifacts and other inherent factors. This application first proposes an approach of monitoring displacement of the surgical instrument by monitoring displacement of the arc-shaped top end portion, and improves structural design of the entire surgical instrument especially the top end portion to improve the identifiability of the region in the ultrasonic image, and further combines with a deep learning algorithm to improve accuracy of identifying image features of the tracking sub-region and improve computational efficiency of data processing.

The deep learning algorithm generally used in the field of image processing requires a large amount of samples and hardware resources for training. In contrast, this application provides a method for identification and tracking based on a small sample of a tracking sub-region. The method includes: constructing an offline feature template library based on the tracking sub-region; updating and determining a current feature template in real time based on a newly input current frame image; and determining the tracking sub-region in the current frame image according to a predetermined search range and search strategy in the current frame image based on feature information in the current feature template.

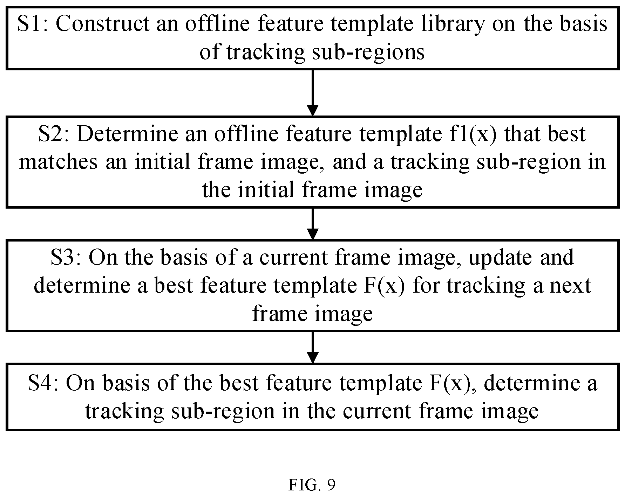

The steps of the method are described in detail below with reference to FIG. 9.

In step S1, an offline feature template library is constructed based on the tracking sub-region.

In step S11, an original feature template of the tracking sub-region is offline acquired.

The original feature template is a combination of the ultrasonic image and feature information of the tracking sub-region, and the feature information is, for example, fHOG features. The offline acquisition method includes, but is not limited to the following: acquiring an image of a surgical instrument including an arc-shaped top end portion using an ultrasonic probe before surgery and extracting feature information thereof; or pre-providing an image of a surgical instrument including an arc-shaped top end portion and extracting feature information thereof.

In step S12, an offline feature template library is constructed based on the original feature template. The offline feature template library includes a plurality of feature templates generated based on the original feature template. The offline feature template library may be constructed by the following steps.

In S121, for the same target field of view or target tissue site, there is a difference in pixel scale between ultrasonic images acquired by ultrasonic probes with different depth parameters. Therefore, the original feature template is expanded to feature templates under different depth parameters, which may be applied to scenarios with different depth parameters, thereby effectively improving accuracy of image feature matching calculation. Taking the scenario of waterjet surgery on prostate tissue as an example, feature templates at depth parameters of 39 mm, 47 mm, 55 mm and 63 mm can be extracted to construct a feature template library applicable to different depth parameters.

In S122, when the sheath and the waterjet are inserted into the target tissue site, there may be an angular deviation or scale. Therefore, n feature templates with different scales and angles are generated based on the original feature template and a preset scale factor to construct a feature template library that is applicable to different scales and angles, thereby effectively improving accuracy of image feature matching calculation.

Taking the scenario of waterjet surgery on prostate tissue as an example, unlike general target tracking, after the waterjet is inserted into the target tissue site, the sheath is usually required to be basically level with the ultrasonic probe. Therefore, the scale of the waterjet in the ultrasonic image does not change significantly when an ultrasonic depth is fixed. The main reason for the scale change is caused by switching of the ultrasonic depth parameter rather than the depth of the waterjet in the current frame. At the same depth, considering that the ultrasonic probe itself has a certain width, when the waterjet undergoes a certain deflection, its projection onto a two-dimensional plane may change in scale. Therefore, an angle parameter is preferably {0°, ±5°, ±10°}.

Further, the scale factor is determined in a manner of multi-scale sampling under different depth parameters, that is, the scale factor is determined according to the depth parameter, thereby improving computational efficiency and reducing the template sample size. For example, the scale factor R is set to be {0.99, 1.0, 1.01} at a depth of 63 mm, the scale factor R is set to be {0.98, 1.0, 1.02} at a depth of 55 mm, the scale factor R is set to be {0.96, 1.0, 1.04} at a depth of 47 mm, and the scale factor R is set to be {0.95, 1.0, 1.05} at a depth of 39 mm. The scale factors are set according to experimental effects at different depths during design.

Based on the steps S121 and S122, a feature template library applicable to different depths, scales and angles can be obtained. The best feature templates for scenarios with different depth, scale and angle parameters can be determined, so as to eliminate unnecessary error factors in subsequent similarity matching calculation, thereby enabling the calculated results to more accurately reflect objective positional deviation information. In particular, after numerous experiments, the inventors of this application have proposed the above parameter selection manner for the scenario of waterjet surgery on prostate tissue, which can achieve a moderate expansion of the original feature template. The constructed offline feature template library has a moderate amount of data, improving calculation accuracy while also taking into account computational efficiency.

In step S2, an offline feature template f1(x) that best matches the initial frame image, and the tracking sub-region in the initial frame image are determined.

After the ultrasonic probe and the surgical instrument are in place, the ultrasonic probe starts to acquire a series of ultrasonic image frames. The acquired first ultrasonic image is referred to as an initial frame image.

In step S21, a plurality of candidate feature templates corresponding to the depth parameter are selected from the offline feature template library based on the depth parameter of the ultrasonic probe. The plurality of candidate feature templates are feature templates based on different angle parameters and different scale factors.

In step S22, a matching degree between the initial frame image and each of the plurality of candidate feature templates is calculated one by one, a feature template having a highest matching degree is taken as the best matching offline feature template f1(x), and a region having a highest matching degree is taken as the tracking sub-region in the initial frame image. Since x is the image of the tracking sub-region, when selecting the best offline feature template f1(x), the tracking sub-region in the initial frame image is also determined correspondingly.

In some cases (for example, when the computational load is not significantly different), step S21 may also be omitted and the best matching offline feature template f1(x) can be directly obtained by executing step S22.

In step S3, based on the current frame image, the best feature template f(x) for tracking a next frame image is updated and determined.

After the tracking starts, a series of ultrasonic image frames of the target tissue site are continuously acquired by the ultrasonic probe and transmitted to the processor. Due to the inherent poor image quality of images acquired by the ultrasonic probe, there may be a difference in contrast or interference from unknown artifacts on the current frame image. Therefore, search and matching operations based solely on the feature template f1(x) in the offline feature template library are not effective, and it is necessary to further correct the feature template. Thus, starting from an initial frame, the best feature template F(x) applicable for the next frame image is updated and determined according to information on each current frame image, which is used for the search and matching operations of the tracking sub-region in the next frame image.

This application adopts a dynamic feature fusion extraction method, which includes: based on a feature (for example, fHOG feature) of the tracking sub-region in the feature template f1(x) selected from the offline feature template library and a feature (for example, fHOG feature) of the tracking sub-region in the current frame image, deriving the best feature template F(x) for the next frame image by preset-weighted fusion.

Further, not only based on the feature (for example, fHOG feature) of the tracking sub-region in the feature template f1(x) selected from the offline feature template library and the feature (for example, fHOG feature) of the tracking sub-region in the current frame image, but also based on a deep feature of the tracking sub-region in the current frame image (for example, a dimensionality-reduced feature extracted from the Conv4 layer of resnet50 trained on imagenet100) extracted by a pre-trained deep learning neural network, the best feature template F(x) for the next frame image is derived by weighted fusion.

The above-mentioned dynamic feature fusion manner at least has the following advantages: by introducing the feature template f1(x) in the offline feature template library, accumulated errors caused during continuous updating can be effectively avoided; and deep semantic information of the tracking sub-region in the current frame image is extracted by a pre-trained deep learning neural network to effectively improve stability of the extracted image features.

In step S4, the tracking sub-region in the current frame image is determined based on the best feature template F(x).

A current frame image is acquired to search in the current frame image according to a set strategy so as to find a region with a maximum response value to the best feature template F(x), that is, the tracking sub-region in the current frame image.

A preferred embodiment of this application further provides a tracking scheme that can adaptively adjust a window size of the tracking sub-region in a waterjet surgery scenario. Different from general target tracking problems, the scale of the waterjet in the ultrasonic image does not change significantly when the ultrasonic depth is fixed. The main reason for the scale change is caused by switching of the ultrasonic depth parameter rather than the depth of a target object in the current frame. At the same depth, considering that the ultrasonic probe itself has a certain width, when the target object undergoes a certain deflection, its projection onto a two-dimensional plane may change in scale. Therefore, in the embodiment, a current ultrasonic depth parameter is acquired in real time and determined, and when the depth parameter changes between two adjacent frames of ultrasonic images, scale factors in a parameter set are adaptively updated according to a scale factor set corresponding to a set depth. The scale factor set is calculated according to a current switching depth, a target depth, and a correspondence of scale coefficients at different depths in the following table. The depth coefficients used frequently during waterjet surgery, such as 39 mm, 47 mm, 55 mm and 63 mm, are selected for depth test calculation to acquire the corresponding scale coefficients, thereby obtaining a depth-scale coefficient index table as shown below:

| Depth (mm) | Scale coefficient |
|---|---|
| 32 | 1.84 |
| 39 | 1.52 |
| 47 | 1.30 |
| 55 | 1.13 |
| 63 | 1.00 |
| 71 | 0.90 |
| 79 | 0.82 |
| 87 | 0.74 |

A scale factor R = {delta_size, 1, 1/delta_size} is defined, where delta_size = (current ultrasonic depth scale coefficient)/(post-switching ultrasonic depth target scale coefficient). For example, if the ultrasonic depth in the current field of view is 47 mm and the post-switching ultrasonic depth is 55 mm, the current ultrasonic depth scale coefficient in an index table is 1.30, the post-switching ultrasonic depth target scale coefficient is 1.13, and delta_size = 1.30/1.13 = 1.14, so that the scale factor R = {0.87, 1, 1, 14}. By selecting a scale factor corresponding to a depth parameter, all scale factors are multiplied by a window size of an original tracking sub-region to obtain a new window size of the tracking sub-region. The new window size of the tracking sub-region is passed through a filter H to calculate the maximum response value, thereby obtaining an optimal scale, and the window size of the tracking sub-region is updated in the next iteration.

In the tracking scheme for adaptively adjusting the window size of the tracking sub-region, when a depth parameter between two adjacent frames of ultrasonic images changes, the best offline feature template f1(x) also needs to be updated into a new window size. The update step is the same as that in the above-mentioned Step S2. Correspondingly, F(x) is also updated into the adjusted window size of the tracking sub-region.

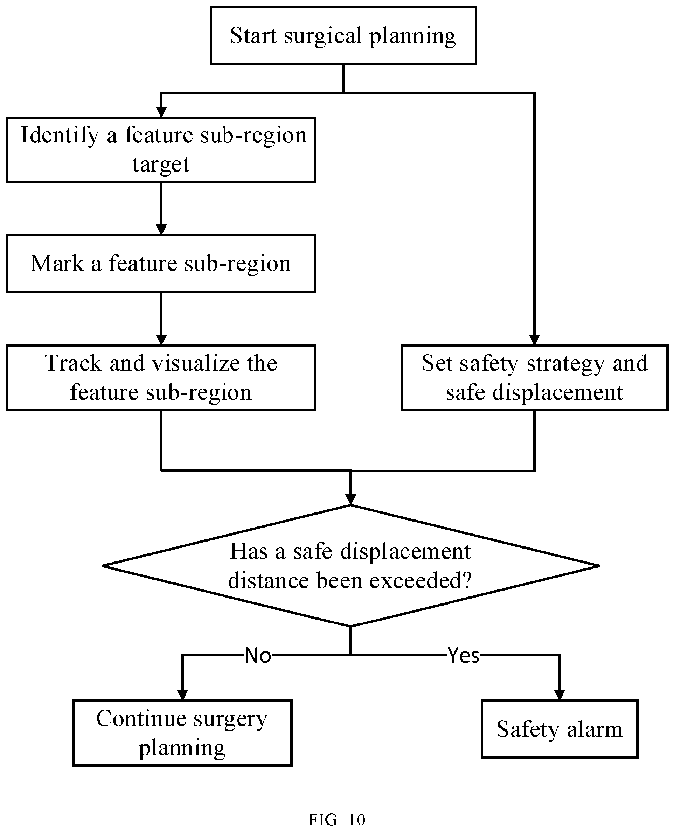

As shown in FIG. 10, when the apparatus for monitoring displacement of the surgical instrument provided by this application is used for surgical planning, the steps of a surgical planning scheme are as follows. As shown in the figure, after the surgery planning starts, a safety strategy corresponding to the displacement distance is set. Meanwhile, an ultrasonic image is identified and a tracking sub-region is marked. Tracking is performed based on the feature of the tracking sub-region, and the tracking information is visualized. When it is determined that the displacement distance exceeds the safe displacement distance based on the tracking information, an alarm is triggered; otherwise, the surgery planning continues.

The inventive concept of this application is not limited to the prostate tissue surgery, and can be applied in other similar surgical environments. The similar surgical environment refers to the environment where the ultrasonic probe is configured to acquire an ultrasonic image including a target tissue site where a surgical instrument is located in real time, that is, the surgical instrument inserted into the target tissue site should be located within the field of view of the ultrasonic probe. Further, although the waterjet is taken as an example in some embodiments, it should be appreciated that the inventive concept of this application is not limited to the waterjet. When displacement of a surgical instrument (for example, an electric scalpel or a laser ablation device) driven and controlled by a surgical robot or a mechanical arm and moved based on a surgical planning path for performing surgery needs to be monitored, the ultrasonic image-based monitoring scheme proposed in this application may also be used.

The embodiment according to this application can solve the above-mentioned technical problem and has at least one of the following technical effects. The technical scheme in the embodiment can overcome a technical difficulty in monitoring displacement of a surgical instrument based on an ultrasonic image, so that accurate, real-time and effective monitoring of displacement of the surgical instrument based on the ultrasonic image becomes possible. Compared with conventional surgical instrument displacement monitoring methods, the technical scheme significantly reduces costs and eliminates cumbersome operation steps such as disinfecting additional markers. The displacement of the surgical instrument can be monitored using an intraoperative ultrasonic imaging subsystem built-into a surgical system, and monitoring results can be fed back and prompted in real time, facilitating the operation for an operator or a doctor. Meanwhile, accurate and real-time displacement monitoring facilitates the safe implementation of a surgical scheme in strict accordance with the surgical planning, improves the quality of surgery, and reduces medical accidents. In addition, the displacement monitoring information may also be directly used to adjust a surgical planning scheme, which is beneficial for precise control of an automated surgical procedure.

The foregoing descriptions are merely the embodiments of this application, and are not intended to limit this application. For those skilled in the art, various modifications and variations may be made to this application. Any modifications, equivalent substitutions and improvements made within the spirit and principles of this application shall be included in the scope of the claims of this application.

## Claims

1. An ultrasonic image-based apparatus for monitoring positional deviation of a surgical instrument, the apparatus comprising: a surgical instrument; an ultrasonic subsystem including an ultrasonic probe; and a control subsystem including a processor, the control subsystem being separately connected to the surgical instrument and the ultrasonic subsystem, the surgical instrument moving in a target tissue site according to a surgical planning path set by the processor, wherein
a tail end of the surgical instrument is provided with an arc-shaped top end portion including a curved outer surface and a curved inner surface, the inner surface is an arc-shaped surface relatively closer to a jetting region of the surgical instrument, and the outer surface is an arc-shaped surface relatively farther from the jetting region of the surgical instrument,
after the surgical instrument is in place, the ultrasonic probe starts to continuously acquire ultrasonic images of the target tissue site and transmit acquired series of ultrasonic image frames to the processor of the control subsystem, the processor includes an image processing program configured to perform calculation based on the series of ultrasonic image frames to determine a positional deviation between a tracking sub-region in a current frame image and a tracking sub-region in a previous frame image, the tracking sub-region is a local region including the arc-shaped top end portion in the ultrasonic images, and the image processing program extracts image features of the tracking sub-region by a feature extraction method to obtain image features of the arc-shaped top end portion for subsequent displacement tracking, and
the surgical instrument includes a waterjet and a sheath including an elongated support body, and the arc-shaped top end portion extends from a distal end of the elongated support body, or the surgical instrument includes a waterjet including an elongated cutter tube and a cutter head located at a distal end of the cutter tube, and the cutter head has an arc shape and forms the arc-shaped top end portion.

2. The apparatus for monitoring positional deviation of a surgical instrument according to claim 1, further comprising: a fixed base connected to the surgical instrument through a first mechanical arm and a first adapter and connected to the ultrasonic probe through a second mechanical arm and a second adapter, the ultrasonic probe being a transrectal ultrasonic probe.

3. The apparatus for monitoring positional deviation of a surgical instrument according to claim 1, wherein the inner surface and the outer surface together form an arc-shaped top end portion with a bending angle of 30° to 75°.

4. The apparatus for monitoring positional deviation of a surgical instrument according to claim 1, wherein the arc-shaped top end portion comprises tungsten, and/or the arc-shaped top end portion comprises a smooth outer surface and a rough inner surface, and the rough inner surface is formed by embedding irregular concave-convex pits, grooves, or steps, or by frosting.

5. The apparatus for monitoring positional deviation of a surgical instrument according to claim 1, wherein a distance between the ultrasonic probe and the sheath is R, and a curvature of the arc-shaped top end portion is K=1/R.

6. The apparatus for monitoring positional deviation of a surgical instrument according to claim 1, wherein the tracking sub-region is a smallest image region including all arc-shaped top end portions.

7. The apparatus for monitoring positional deviation of a surgical instrument according to claim 1, wherein the apparatus alarms or pauses a surgery when the monitored positional deviation exceeds a set safety displacement distance.

8. The apparatus for monitoring positional deviation of a surgical instrument according to any one of claims 1 to 7, wherein the image processing program further performs steps of: identifying and marking a tracking sub-region on an initial frame image; updating the tracking sub-region in each subsequent frame image; and calculating a positional deviation between the tracking sub-region in the current frame image and an estimated tracking sub-region, where a position of the estimated tracking sub-region is a position of the tracking sub-region in the previous frame image or a position obtained by superimposing a planned movement path between two frames onto the position of the tracking sub-region in the previous frame image.

9. The apparatus for monitoring positional deviation of a surgical instrument according to any one of claims 1 to 7, wherein the image processing program further performs steps of: setting center position coordinates of the tracking sub-region on the initial frame image as an original coordinate origin for tracking; subsequently updating the tracking sub-region in each subsequent frame image and calculating center position coordinates of the updated tracking sub-region; and calculating a deviation between center position coordinates of the tracking sub-region in the current frame image and center position coordinates of the estimated tracking sub-region.

10. The apparatus for monitoring positional deviation of a surgical instrument according to any one of claims 1 to 7, wherein the image processing program further displays boundary points or center position points of the tracking sub-region in the current frame image in different colors and/or prompts textual information thereof based on a magnitude of the positional deviation.

11. The apparatus for monitoring positional deviation of a surgical instrument according to any one of claims 1 to 7, wherein the image processing program performs identification and tracking based on a small sample of the tracking sub-region.

12. The apparatus for monitoring positional deviation of a surgical instrument according to any one of claims 1 to 7, wherein the image processing program further performs steps of: constructing an offline feature template library based on the tracking sub-region; determining an offline feature template f1(x) that best matches the initial frame image and the tracking sub-region in the initial frame image; determining a best feature template F(x) for a next frame image according to each current frame image; and determining a tracking sub-region in the next frame image based on the determined best feature template F(x).

13. The apparatus for monitoring positional deviation of a surgical instrument according to claim 12, wherein the step of constructing an offline feature template library based on the tracking sub-region includes a step of offline acquiring an original feature template of the tracking sub-region, the original feature template being a combination of ultrasonic images and feature information of the tracking sub-region.

14. The apparatus for monitoring positional deviation of a surgical instrument according to claim 13, wherein the step of constructing an offline feature template library based on the tracking sub-region further includes a step of constructing an offline feature template library based on the original feature template, the offline feature template library including a plurality of feature templates generated based on the original feature template.

15. The apparatus for monitoring positional deviation of a surgical instrument according to claim 14, wherein the offline feature template library includes offline feature templates with different depth parameters and offline feature templates of different scales formed by determining scale factors based on the different depth parameters.

16. The apparatus for monitoring positional deviation of a surgical instrument according to claim 12, wherein the step of determining an offline feature template f1(x) that best matches the initial frame image and the tracking sub-region in the initial frame image includes: after both the ultrasonic probe and the surgical instrument are in place, starting the ultrasonic probe to acquire the initial frame image; selecting, based on a depth parameter of the ultrasonic probe, a plurality of candidate feature templates corresponding to the depth parameter from the offline feature template library; and calculating a matching degree between the initial frame image and each of the plurality of candidate feature templates one by one, taking a feature template having a highest matching degree as the best matching offline feature template f1(x), and taking a region having a highest matching degree as the tracking sub-region in the initial frame image.

17. The apparatus for monitoring positional deviation of a surgical instrument according to claim 12, wherein in the step of determining a best feature template F(x) for a next frame image according to each current frame image, the best feature template F(x) for the next frame image is determined at least based on an image feature of the tracking sub-region in the feature template f1(x) and an image feature of the tracking sub-region in the current frame image.

18. The apparatus for monitoring positional deviation of a surgical instrument according to claim 17, wherein in the step of determining a best feature template F(x) for each next frame image based on each current frame image, based on a feature of the tracking sub-region in the feature template f1(x) selected from the offline feature template library, a feature of the tracking sub-region in the current frame image, and a deep feature of the tracking sub-region in the current frame image extracted by a pre-trained deep learning neural network, the best feature template F(x) for the next frame image is derived by weighted fusion.

19. The apparatus for monitoring positional deviation of a surgical instrument according to claim 12, wherein a window size of the tracking sub-region is adaptively adjustable.

20. The apparatus for monitoring positional deviation of a surgical instrument according to claim 19, wherein a current ultrasonic depth parameter is acquired in real time and determined, and when the depth parameter changes between two adjacent frames of ultrasonic images, scale factors in a parameter set are adaptively updated according to a scale factor set corresponding to a set depth.
